# EUROPEAN PATENT APPLICATION

(11) **EP 4 428 146 A1**
(43) Date of publication of application: **11.09.2024**
(21) Application number: 21962927.6
(22) Date of filing: 05.11.2021
(51) Int. Cl.: C07K 14/475, C07K 1/22, C07K 1/16, C12N 15/12, C12N 15/85, A61K 48/00, A61K 39/00, A61K 38/18, A61K 47/64, A61K 45/06, A61P 7/02

(54) **USE OF PROTEIN SEQUENCE CAPABLE OF BINDING TO SUBSTRATE IN PREPARATION OF PRODUCT FOR INHIBITING FIBRIN ASSEMBLY**

(30) Priority: 02.11.2021 CN 202111290823
(71) Applicant: Nanjing University, Nanjing, Jiangsu 210023 (CN); Center for Excellence in Molecular Cell Science, Chinese Academy of Sciences, Shanghai 200031 (CN); Huazhong Agricultural University, Wuhan, Hubei 430070 (CN)
(72) Inventor: DONG, Xianchi, Nanjing, Jiangsu 210023 (CN); LIU, Wen, Nanjing, Jiangsu 210023 (CN); DING, Jianping, Shanghai 200031 (CN); SHI, Yu, Shanghai 200031 (CN); XU, Shutong, Wuhan, Hubei 430070 (CN); XU, Jianbo, Wuhan, Hubei 430070 (CN)
(74) Representative: Garavelli, Paolo
(86) International application number: PCT/CN2021/128944
(87) International publication number: WO 2023/077413

(57) **Abstract**

Provided are a protein containing an amino acid sequence capable of binding to a peptide fragment of a substrate GPRP, a fibrinogen-like protein 1 (FGL1) containing the amino acid sequence, and a fibrinogen domain (FD) of the FGL1, wherein a single FD protein also has a function the same as or similar to that of the FGL1. The FGL1 and FD, which have different action mechanisms from existing drugs, can inhibit fibrin assembly in the process of thrombogenesis by means of competing for fibrin substrate binding pockets. The FGL1 and FD can have a stronger affinity to the substrate by means of mutation, thereby effectively enhancing a usage effect of drugs for treating thrombus.

## Description

### Field

The present invention relates to a protein sequence capable of binding to a substrate, fibrinogen-like protein 1, which comprises the protein sequence, and the application of the active structural domain thereof in preparing products that inhibit fibrin assembly.

### BACKGROUND OF THE INVENTION

A thrombus is a small mass formed by blood flow on the inner surface of a vessel of the cardiovascular system at the site of denudation or repair. In variable flow-dependent patterns (VFDPs), thrombi consist of insoluble fibrin, deposited platelets, accumulated leukocytes, and trapped red blood cells. Thrombosis is a multifactorial process of change in which a set of genetic and environmental factors interact and influence each other. The most important characteristics of patients with common clinical thrombosis are familial inheritance, recurrent episodes, the severity of symptoms, abnormal site of thrombosis, and youthful onset. Antithrombotic therapy involves using thrombolytic drugs, antiplatelet drugs, and anticoagulants to inhibit the formation of blood clots or to break down clots that have already formed.

Antithrombotic drugs can be divided into three categories: anticoagulants, antiplatelet aggregation drugs and thrombolytic drugs:
A. Anticoagulants are a class of drugs that interfere with coagulation factors and prevent blood clotting. They are mainly used in the prevention and treatment of thromboembolic diseases.
B. Antiplatelet-aggregating drugs are divided into three generations: aspirin is a drug of the first generation, ticlopidine is a drug of the second generation, and platelet glycoprotein IIb/IIIa receptor antagonist is a drug of the third generation. Among them, the appearance of platelet glycoproteinIIb/IIIa receptor antagonists is an essential milestone in antiplatelet therapy.
C. Fibrin formed in coagulation can be decomposed by the function of fibrinolytic enzyme from the arginine-lysine bond into soluble products to dissolve the thrombus. Fibrinolytic drugs activate fibrinolytic enzymes and promote fibrinolysis. They are also known as thrombolytic drugs and are used in treating acute thromboembolic diseases. The first generation of thrombolytic drugs, streptokinase (SK) and urokinase (UK), are still the most widely used at home and abroad, and with the appearance of the new generation of thrombolytic drugs, such as Pro-urokinase (Pro-UK), this type of drug is being gradually popularized in the clinic.

Fibrinogen (also known as plasma fibrinogen) is a glycoprotein (α2β2γ2) synthesized and secreted by liver cells; vital protein fibrin is involved in coagulation and hemostasis. It is a monomeric protein generated by the thrombin excision of fibrinopeptides A and B in plasma fibrinogen during coagulation. Hyperfibrinogen is a significant risk factor for various thrombotic diseases and is considered a marker of a disease state in clinical practice.

### SUMMARY OF THE INVENTION

At least one specification heading is required. Please delete this heading section if it is not applicable to your application. For more information regarding the headings of the specification, please see MPEP 608.01(a).

### BRIEF DESCRIPTION OF DRAWINGS

To more clearly illustrate the technical solutions in the embodiments of the present invention, the accompanying drawings to be used in the description of the embodiments will be briefly described below, and it will be evident that the accompanying drawings in the following description are only shown some of the embodiments of the present invention, and that for a person of ordinary skill in the art, other accompanying drawings can be obtained based on these drawings without creative labour.
Figure 1 shows an SDS-PAGE photograph of fibrinogen-like protein 1 (FGL1) after Ni-NTA affinity column purification.
Figure 2 shows an elution graph of Fibrinogen like protein 1 (FGL1) after purification on a Superdex 200 10/300 GL chromatography column. The vertical coordinate of the graph is the A280 protein absorption value, and the horizontal coordinate is the number of volumes.
Figure 3 shows the SDS-PAGE photograph of fibrinogen-like protein 1 (FGL1) purified by Superdex 200 10/300 GL chromatography column; wherein lanes 1-12 are individually shown as: 1-protein standard, 2-collection tube 1, 3-collection tube 2, 4-collection tube 3, 5-collection tube 4, 6-collection tube 5, 7-collection tube 6, 8-collection tube 7, 9-collection tube 8, 10-collection tube 9, 11-collection tube 10, 12-protein standard.
Figure 4 shows the SDS-PAGE photograph of fibrinogen structural domain (FD) after purification on Ni-NTA affinity column.
Figure 5 shows a graph of the fibrinogen structural domain (FD) purified by the Superdex 200 10/300 GL chromatography column, wherein the vertical coordinate is the A280 protein absorption value and the horizontal coordinate is the number of volumes.
Figure 6 shows the SDS-PAGE photograph of the fibrinogen structural domain (FD) after purification on a Superdex 200 10/300 GL chromatography column; wherein, lanes 19-29 are individually shown as: 19-collection tube 19, 20-collection tube 20, 21-collection tube 21, 22-collection tube 22, 23-collection tube 23, 24-collection tube 24, 25-collection tube 25, 26-collection tube 26, 27-collection tube 27, 28-collection tube 28, and 29-protein standard.
Figure 7 shows the effect of fibrinogen-like protein 1 (FGL1) on fibrin aggregation without calcium chloride.
Figure 8 shows the effect of fibrinogen-like protein 1 (FGL1) on fibrin aggregation in the presence of calcium chloride.
Figure 9 shows the effect of fibrinogen structural domain (FD) on fibrin aggregation without calcium chloride.
Figure 10 shows the effect of fibrinogen structural domain (FD) on fibrin aggregation in the presence of calcium chloride.
Figure 11 shows a comparative graph of the effect of fibrinogen-like protein 1 (FGL1) on fibrin aggregation observed by scanning electron microscopy.
Figure 12 shows a comparative graph of the effect of fibrinogen structural domain (FD) on fibrin aggregation observed by scanning electron microscopy. It can be seen that the thrombus formed by fibrin aggregation becomes thinner and looser in structure under the treatment of fibrinogen structural domain (FD).
Figure 13 shows a comparative graph of the effect of fibrinogen-like protein 1 (FGL1) on thrombus morphology in mice, which was observed by scanning electron microscopy.
Figure 14 shows a comparative graph of the effect of fibrinogen structural domain (FD) on the morphology of mouse thrombus observed by scanning electron microscopy. Under the fibrinogen structural domain (FD) treatment, the thrombus becomes thinner and looser in structure.
Figure 15 shows a control plot of the effect of the action of the fibrinogen structural domain (FD) on the cleavage of aggregated fibrin.
Figure 16 shows a control plot of the effect of action of Fibrinogen like protein 1 (FGL1) on the cleavage of aggregated fibrin.
Figure 17 shows a comparative plot of Fibrinogen like protein 1 (FGL1) synergistically dissolving fibrin aggregates with tissue fibrinogen activator tPA.
Figure 18 shows a comparative plot of fibrinogen structural domain (FD) synergistically dissolving fibrin aggregates with tissue fibrinogen activator tPA.
Figure 19 shows a comparative plot of Fibrinogen like protein 1 (FGL1) synergistically dissolving fibrin aggregates with urokinase uPA.
Figure 20 shows a comparative plot of fibrinogen structural domain (FD) synergistically dissolving fibrin aggregates with urokinase uPA.
Figure 21 shows a statistical graph of the data of Fibrinogen like protein 1 (FGL1) plasmid injected in mice to overexpress for improving thrombosis model induced by thrombin, wherein (A) is a comparative graph of verifying the overexpression of Fibrinogen like protein 1 (FGL1) plasmid injected with ELISA, and (B) is a comparative graph of the overexpression of Fibrinogen like protein 1 (FGL1) for significantly increasing the survival rate of mice.
Figure 22 shows statistical graphs of data from the thrombin-induced thrombus model improved by overexpression of fibrinogen structural domain (FD) plasmid injected in mice; wherein (A) is a comparative graph of validation of overexpression of fibrinogen structural domain (FD) plasmid injected with ELISA; and (B) is a comparative graph of significant improvement of survival rate in mice by overexpression of fibrinogen structural domain (FD).
Figure 23 shows an operational procedure for fibrinogen like protein 1 (FGL1) modified by RVG peptide and Cy5.5 fluorescence.
Figure 24 shows a graph of the results of the distribution assay of Fibrinogen like protein 1 (FGL1) in mouse brain tissue promoted by modification of RVG peptide.
Figure 25 shows that Fibrinogen like protein 1 (FGL1) and the structural domain of fibrinogen structural domain (FD) had no significant effect on the loss of Fibrinogen (A) and the bleeding time (B) (no toxic side effects).
Figure 26 shows a structural diagram of Fibrinogen like protein 1 (FGL1) (green) and substrate GPRP peptide binding (orange); wherein the amino acids shown as markers are the key sites for substrate binding, which individually are tryptophan at position 204, tyrosine at position 222, cysteine at position 226, glutamic acid at position 229, aspartic acid at position 230, cysteine at position 239, histidine at position 240, and aspartic acid at position 258, tyrosine at position 264, and tryptophan at position 269; B of Figure 26 is obtained by rotating A of Figure 26 by 70° along the Y axis.
Figure 27 shows a comparative plot of the cleavage effect of fibrinogen structural domain (FD) mutants on aggregated fibrin; wherein the mutation sites were tryptophan at positions 204 and 269, and the cleavage effect of the mutated fibrinogen structural domain (FD) mutants were significantly enhanced by mutation from tryptophan to alanine.

### DETAILED DESCRIPTION OF THE INVENTION

It is an object of the present invention to provide a protein containing an amino acid sequence that binds to a peptide segment of a substrate GPRP, a fibrinogen like protein 1 (FGL1) containing this amino acid sequence, and a fibrinogen domain of FGL1 (FD) thereof, which has a different mechanism of action from that of the existing drugs and can inhibit fibrin assembly during thrombosis by competing for the binding of the fibrin γ subunit to fibrinopeptide A.

The following technical solutions may realize the above objects of the present invention:

The first point of the present invention is to provide a protein, said protein containing an amino acid sequence that can bind to a substrate, GPRP peptide, the amino acid sequence having the structure of tryptophan-AAₙ₁ -tyrosine-AAₙ₂ -cysteine -AAₙ₃-glutamic acid-aspartic acid-AAₙ₄-cysteine-histidine-AAₙ₅- Aspartic acid -AAₙ₆-tyrosine -AAₙ₇-tryptophan, where AA is an amino acid, n1-n7 are the number of amino acids, n1=16-18, n2=2-4, n3=1-3, n4=7-9, n5= 16-18, n6=4-6, n7=3-5.

The protein as mentioned above, said numbers of amino acids n1 = 17, n2= 3, n3= 2, n4= 8, n5= 17, n6= 5, n7= 4.

That is, the structure of the amino acid sequence is as follows: tryptophan - 17 amino acids-tyrosine-3 amino acids - cysteine - 2 amino acids - glutamic acid -aspartic acid - 8 amino acids - cysteine - histidine - 17 amino acids - aspartic acid - 5 amino acids -tyrosine - 4 amino acids -tryptophan.

It is shown that there are any 17 amino acids connected between tryptophan and tyrosine, any 3 amino acids connected between tyrosine and cysteine, any 2 amino acids connected between cysteine and glutamic acid, a direct connection between glutamic acid and aspartic acid, any 8 amino acids connected between aspartic acid and another cysteine, a direct connection between another cysteine and histidine, any 17 amino acids connected between histidine and another aspartic acid, any 5 amino acids connected between another aspartic acid and another tyrosine, and any 4 amino acids connected between another tyrosine and another tryptophan.

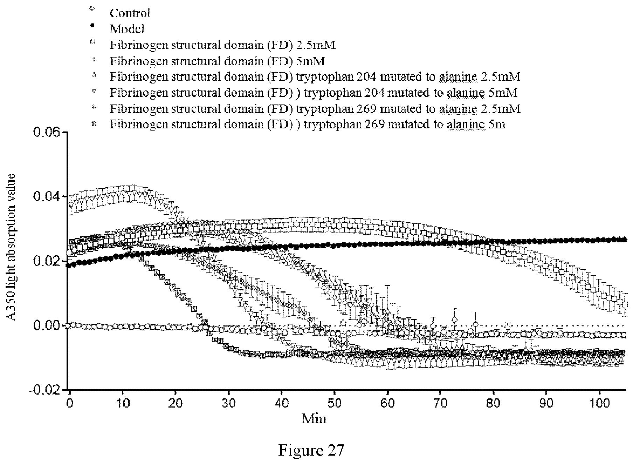

The amino acid sequence contained in this protein can bind to the peptide segment of the substrate GPRP, under the condition that the existence of the key sites of this amino acid sequence as well as the same structure or 80% of the same structure is guaranteed, the AA in the amino acid sequence can be any functional amino acid, and the adoption of AA with different functions will also bring about different compounding efficacies to the said protein; at the same time, the mutation of certain key sites in the amino acid sequence of this protein may also achieve the effect of enhancing its thrombosis inhibition function, such as in the case where tryptophan at position 204 and/or 269 is mutated to alanine, the cleavage of aggregated fibrin by the mutant is significantly enhanced compared to that in the non-mutated form (shown in Figure 27).

Specifically, in the Fibrinogen like protein 1 provided by the present invention, the amino acid sequence comprises tryptophan at position 204, tyrosine at position 222, cysteine at position 226, glutamic acid at position 229, aspartic acid at position 230, cysteine at position 239, histidine at position 240, aspartic acid at position 258, tyrosine at position 264, and tryptophan at position 269.

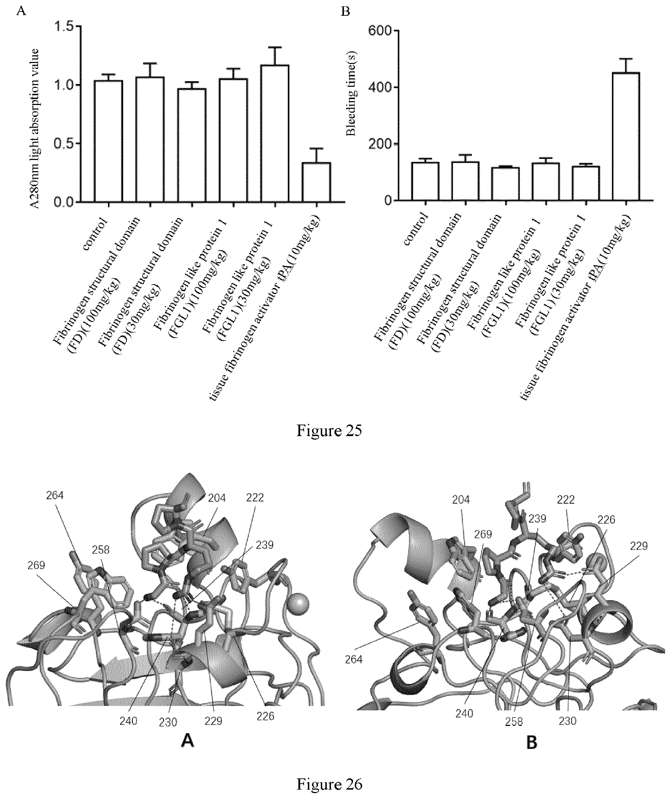

The amino acid sites listed in the above amino acid sequences are key amino acid sites that can bind to the substrate GPRP (Gly-Pro-Arg-Pro) peptide. The amino acid sequences can specifically and competitively bind to the substrate GPRP by hydrophobic bonding, hydrogen bonding, or salt bonding through these key amino acid sites, as shown in Figure 26, which competitively occupy the binding sites of the peptide that can bind to the fibrinopeptide A, thus achieving the effect of inhibiting thrombosis or of slowing down the progress of thrombosis by inhibiting the assembly of fibrin and inhibiting the activity of fibrin.

A second point of the present invention is to provide Fibrinogen like protein 1, being the protein shown in any one of the following items and comprising the amino acid sequence as previously described:
(a1) A protein having an amino acid sequence as shown in SEQ ID No. 1 (i.e., SEQ ID No. 1) or positions 48-290 of SEQ ID No. 1 (i.e., SEQ ID No. 3);
(a2) A protein in which the amino acid sequence shown in SEQ ID No. 1 or positions 48-290 of SEQ ID No. 1 has been subjected to one or several substitutions and/or deletions and/or additions of amino acid residues and has the same functions;
(a3) A protein having more than 99%, more than 95%, more than 90%, more than 85% or more than 80% homology with the amino acid sequence defined in any one of (a1)-(a2) and having the same functions;
(a4) A fusion protein obtained by joining tags at the N-terminus and/or C-terminus of a protein defined in any one of (a1)-(a3).

Fibrinogen like protein 1 (FGL1) comprises a fibrinogen structural domain protein (FD), and the FD structural domain protein alone also has the same or a similar function as FGL1; wherein the amino acid sequence of FGL1 is as shown in SEQ ID No. 1 in the sequence table, and the amino acid sequence of the FD structural domain protein alone is as shown at positions 48-290 in the sequence table in SEQ ID No. 1.

The amino acid sequence of Fibrinogen like protein 1 (FGL1) is shown below, SEQ ID No. 1:

LEDCAQEQMRLRAQVRLLETRVKQQQVKIKQLLQENEVQFLDKGDENTVIDLGS
KRQYADCSEIFNDGYKLSGFYKIKPLQSPAEFSVYCDMSDGGGWTVIQRRSDGSENFNR
GWKDYENGFGNFVQKHGEYWLGNKNLHFLTTQEDYTLKIDLADFEKNSRYAQYKNFK
VGDEKNFYELNIGEYSGTAGDSLAGNFHPEVQWWASHQRMKFSTWDRDHDNYEGNC
AEEDQSGWWFNRCHSANLNGVYYSGPYTAKTDNGIVWYTWHGWWYSLKSVVMKIRP
NDFIPNVI

The amino acid sequence of the Fibrinogen structural domain protein (FD) is shown below, SEQ ID No. 3:

TVIDLGSKRQYADCSEIFNDGYKLSGFYKIKPLQSPAEFSVYCDMSDGGGWTVIQ
RRSDGSENFNRGWKDYENGFGNFVQKHGEYWLGNKNLHFLTTQEDYTLKIDLADFEK
NSRYAQYKNFKVGDEKNFYELNIGEYSGTAGDSLAGNFHPEVQWWASHQRMKFSTWD
RDHDNYEGNCAEEDQSGWWFNRCHSANLNGVYYSGPYTAKTDNGIVWYTWHGWWY
SLKSVVMKIRPNDFIPNVI

A third point of the present invention is the provision of nucleic acid molecules encoding the said Fibrinogen like protein 1.

Further, said nucleic acid molecule is a gene; said gene is a DNA molecule described in any one of the following:
(b1) a DNA molecule is shown in SEQ ID No. 2 (i.e., SEQ ID No. 2) or positions 142-870 of SEQ ID No. 2 (i.e., SEQ ID No. 4);
(b2) A DNA molecule that hybridizes under stringent conditions to a DNA molecule defined by (b1) and encodes said protein;
(b3) A DNA molecule having more than 99%, more than 95%, more than 90%, more than 85%, or more than 80% homology to a DNA sequence defined in (b1)-(b2) and encoding said protein.
(b4) A DNA molecule encoding said protein described in (a3).

The nucleic acid sequence of Fibrinogen like protein 1 (FGL1) is shown below, SEQ ID No. 2:

ctcgaggactgtgcccaggagcagatgcggctcagagcccaggtgcgcctgcttgagacccgggtcaaacagcaacaggtc
aagatcaagcagcttttgcaggagaatgaagtccagttccttgataaaggagatgagaatactgtcattgatcttggaagcaagaggcagtat
gcagattgttcagagattttcaatgatgggtataagctcagtggattttacaaaatcaaacctctccagagcccagcagaattttctgtttattgtg
acatgtccgatggaggaggatggactgtaattcagagacgatctgatggcagtgaaaactttaacagaggatggaaagactatgaaaatgg
ctttggaaattttgtccaaaaacatggtgaatattggctgggcaataaaaatcttcacttcttgaccactcaagaagactacactttaaaaatcga
ccttgcagattttgaaaaaaatagccgttatgcacaatataagaatttcaaagttggagatgaaaagaatttctacgagttgaatattggggaat
attctggaacagctggagattcccttgcggggaattttcatcctgaggtgcagtggtgggctagtcaccaaagaatgaaattcagcacgtgg
gacagagatcatgacaactatgaagggaactgcgcagaagaagatcagtctggctggtggtttaacaggtgtcactctgcaaacctgaatg
gtgtatactacagcggcccctacacggctaaaacagacaatgggattgtctggtacacctggcatgggtggtggtattctctgaaatctgtgg
ttatgaaaattaggccaaatgattttattccaaatgtaatt

The nucleic acid sequence of the Fibrinogen structural domain protein (FD) is shown below, SEQ ID No. 4:

actgtcattgatcttggaagcaagaggcagtatgcagattgttcagagattttcaatgatgggtataagctcagtggattttacaaaa
tcaaacctctccagagcccagcagaattttctgtttattgtgacatgtccgatggaggaggatggactgtaattcagagacgatctgatggcag
tgaaaactttaacagaggatggaaagactatgaaaatggctttggaaattttgtccaaaaacatggtgaatattggctgggcaataaaaatctt
cacttcttgaccactcaagaagactacactttaaaaatcgaccttgcagattttgaaaaaaatagccgttatgcacaatataagaatttcaaagtt
ggagatgaaaagaatttctacgagttgaatattggggaatattctggaacagctggagattcccttgcggggaattttcatcctgaggtgcagt
ggtgggctagtcaccaaagaatgaaattcagcacgtgggacagagatcatgacaactatgaagggaactgcgcagaagaagatcagtctg
gctggtggtttaacaggtgtcactctgcaaacctgaatggtgtatactacagcggcccctacacggctaaaacagacaatgggattgtctggt
acacctggcatgggtggtggtattctctgaaatctgtggttatgaaaattaggccaaatgattttattccaaatgtaatt

A fourth point of the present invention is the provision of expression cassettes, recombinant vectors, recombinant bacteria, or transgenic cell lines comprising said nucleic acid molecules.

A fifth point of the present invention is the provision of an application of the above-described Fibrinogen like protein 1 (FGL1) or of the above-described nucleic acid molecules or the above-described recombinant vectors, recombinant bacteria, or transgenic cell line, said application being at least any one of the following:
(c1) preparation of products for inhibiting thrombogenesis;
(c2) Preparation of products for inhibiting the extent of thrombosis;
(c3) Preparation of products for inhibiting fibrin activity in plasma;
(c4) Preparation of products used to inhibit fibrinogen assembly in plasma;
(c5) Preparation of products used to enhance fibrinolytic capacity;
(c6) Preparation of products for prolonging activated partial thromboplastin time of the plasma and/or prothrombin time and/or thrombin time.

The studies of the present invention show that Fibrinogen like protein 1 (FGL1) or fibrinogen structural domain protein (FD) inhibits fibrin assembly during thrombosis, and can be used to prepare a drug for treating thrombosis-related diseases.

Wherein said drug for treating a thrombosis-related disease is a drug having Fibrinogen like protein 1 (FGL1) or fibrinogen structural domain protein (FD) as an active ingredient. Said drug for the treatment of thrombosis-related disorders can be used for the treatment of thrombotic diseases caused by a variety of reasons, as well as for the prevention of thrombotic diseases, at the same time, and for the prevention and treatment of a variety of disorders associated with increased fibrin activity, decreased fibrinolytic capacity, and shortening of the plasma's activated partial thromboplastin time and/or plasminogen time and/or prothrombin time.

Fibrinogen like protein 1 (FGL1) comprises Fibrinogen structural domain proteins (FDs), and its mechanism of action is that it can inhibit fibrin assembly during thrombosis by competing for binding of the fibrin γ subunit to fibrinopeptide A.

Fibrinogen like protein 1 (FGL1) may also be mutated to increase its affinity for fibrinopeptide A, thereby improving the drug's efficacy.

Further, for the above application, the method of preparation of said protein is to clone the gene encoding said protein into a eukaryotic expression vector, to culture eukaryotic cells to express said protein in serum-free medium, purify the protein by using a Ni-NTA affinity column, and then further purify the protein by gel filtration using a Superdex 200 10/300 GL chromatographic column, to obtain a highly purified protein.

Further, with the above application, said product is a pharmaceutical product, said pharmaceutical product comprising a vaccine product.

Further, with the above application, said type of drug product comprises a DNA drug, an mRNA drug, a protein drug, or an adenovirus drug. Said drug product for the prevention or treatment of thrombotic diseases, including but not limited to the types as mentioned above of products, which may be administered in various forms of carriers, such as plasmid encapsulation, microsphere encapsulation, exosome carrying, etc., and may also be administered in multiple forms of protein-carrying delivery modes, such as protein-compound conjugation delivery, protein-peptide conjugation delivery, protein-small-molecule-drug conjugation delivery, etc., which ultimately aim to ensure the biological activity of said Fibrinogen like protein 1 (FGL1) or said fibrinogen structural domain protein (FD) described in the present invention, and ingesting the active ingredient through different delivery methods to inhibit the assembly of fibrin.

Further, with the above application, said product is modified with a structure for crossing the blood-brain barrier.

Further, with the above application, said structure modified for crossing the blood-brain barrier is a brain-targeting ligand.

Further, in said application, the brain-targeting ligand includes but is not limited to, RVG peptide, stearic acid-transferrin, and carrier protein E.

Based on the above-described description, on the premise of ensuring the biological activity of fibrinogen like protein 1 (FGL1) or fibrinogen structural domain protein (FD), the said pharmaceutical product for preventing or treating thrombotic disease, can also be combined with a variety of targeting ligands, and a more prominent targeting ligand modification is to combine the protein or the structural domain protein with the brain-targeting ligand to facilitate its breakthrough of the blood-brain barrier (BBB), thereby realizing supply of drugs to the organism for treatment for brain thrombosis. However, the present invention is not limited to this, as it can likewise be combined with other structures or forms of targeting ligands, thereby realizing targeted therapy for different lesions.

Further, in the above application, said drug also comprises a therapeutic agent that can be combined.

Further, in the above application, said combinable therapeutic agents are urokinase, streptokinase, alteplase, reteplase or tissue fibrinogen activator, respectively.

Fibrinogen like protein 1 (FGL1) and its mutants can effectively synergize with existing antithrombotic drugs (urokinase and tissue fibrinogen activator, etc.) and reduce the dose of existing antithrombotic drugs and alleviate their toxic side effects.

A sixth point of the present invention is to provide a biomaterial; said biomaterial is a nucleic acid molecule capable of expressing said Fibrinogen like protein 1 (FGL1) or an expression cassette, a recombinant vector, a recombinant bacterium, or a transgenic cell line containing said nucleic acid molecule.

Currently, available antithrombotic drugs can be categorized into three groups according to different mechanisms of action, which respectively are:
1) Anticoagulant drugs: the mechanism is to inhibit the coagulation process, and the side effect is to cause tissue bleeding easily;
(2) Antiplatelet drugs: the mechanism is to inhibit platelet aggregation, and the side effect is to cause tissue bleeding;
3) Fibrinolytic drugs: the mechanism is to dissolve the thrombus already formed by inducing fibrin degradation, with the same side effect of bleeding complications.

The features and advantages of the present invention are: the present invention provides proteins containing an amino acid sequence that can bind to a peptide fragment of the substrate GPRP, Fibrinogen like protein 1 (FGL1) containing this amino acid sequence, and its active structural domain (FD), and the application of the proteins mentioned above in the preparation of a product for inhibiting the assembly of fibrin, said proteins containing an amino acid sequence that can bind to the peptide fragment of the substrate GPRP, Fibrinogen like protein 1 (FGL1), its fibrinogen structural domain (FD), and mutants thereof, which have different mechanisms of action from existing drugs, can inhibit fibrin assembly during thrombosis by competing for the fibrin substrate-binding pocket, and through mutation, can be made to have a stronger affinity for the substrate, which can effectively enhance the effectiveness of the use of thrombosis therapy drugs. The mechanism of action of FGL1 and FD is to destroy the assembly process of fibrin, so it has an excellent thrombolytic effect compared with the existing thrombolytic drugs. However, the thrombolytic process is gentler and does not produce side effects like bleeding. That is to say, the present invention is to act on fibrin, not Fibrinogen, which also avoids the technological problem caused by the existing thrombosis therapy drug in the use of the process due to the action on Fibrinogen, which leads to very easily cause bleeding as a side effect of the drug; it is also due to this mechanism of action, the present invention will provide the FGL1 and the FD and a variety of existing thrombosis therapy drugs (such as alteplase, etc.) when used in conjunction, can synergistically treat thrombotic diseases, and at the same time, it can also substantially reduce the incidence of the prevalent bleeding side effects of the existing antithrombotic drugs, expanding a new avenue for the treatment of thrombotic diseases.

### Embodiments

The technical solutions in the examples of the present invention will be clearly and completely described below in conjunction with the accompanying figures in the examples of the present invention, and it is clear that the described embodiments are only a part and not all of the examples of the present invention. Based on the examples in the present invention, all other examples obtained by the person skilled in the art without making creative labour fall within the scope of protection of the present invention.

### Example 1:

A protein, the protein containing an amino acid sequence that can bind to a substrate, GPRP peptide, the amino acid sequence having the structure of: tryptophan-AAₙ₁ -tyrosine-AAₙ₂ -cysteine -AAₙ₃-glutamic acid-aspartic acid-AAₙ₄-cysteine-histidine-AAₙ₅- Aspartic acid -AAₙ₆-tyrosine -AAₙ₇-tryptophan, where AA is an amino acid, n1-n7 are the number of amino acids, n1=16-18, n2=2-4, n3=1-3, n4=7-9, n5= 16-18, n6=4-6, n7=3-5.

Preferably, the number of amino acids n1 = 17, n2= 3, n3= 2, n4= 8, n5= 17, n6= 5, n7= 4.

That is, the structure of the amino acid sequence is as follows: tryptophan - 17 amino acids - tyrosine - 3 amino acids - cysteine - 2 amino acids - glutamic acid -aspartic acid - 8 amino acids - cysteine - histidine - 17 amino acids - aspartic acid - 5 amino acids -tyrosine - 4 amino acids -tryptophan.

It is shown that there are any 17 amino acids connected between tryptophan and tyrosine, any 3 amino acids connected between tyrosine and cysteine, any 2 amino acids connected between cysteine and glutamic acid, a direct connection between glutamic acid and aspartic acid, any 8 amino acids connected between aspartic acid and another cysteine, a direct connection between another cysteine and histidine, any 17 amino acids connected between histidine and another aspartic acid, any 5 amino acids connected between another aspartic acid and another tyrosine, and any 4 amino acids connected between another tyrosine and another tryptophan.

### Example 2:

fibrinogen like protein 1, being the protein shown in any one of the following items and containing the amino acid sequence as previously described in example 1:
(a1) A protein having an amino acid sequence as shown in SEQ ID No. 1 or positions 48-290 of SEQ ID No. 1;
(a2) A protein in which the amino acid sequence shown in SEQ ID No. 1 or positions 48-290 of SEQ ID No. 1 has been subjected to one or several substitutions and/or deletions and/or additions of amino acid residues and has the same functions;
(a3) A protein having more than 99%, more than 95%, more than 90%, more than 85% or more than 80% homology with the amino acid sequence defined in any one of (a1)-(a2) and having the same functions;
(a4) A fusion protein obtained by joining tags at the N-terminus and/or C-terminus of a protein defined in any one of (a1)-(a3).

Fibrinogen like protein 1 (FGL1) comprises a fibrinogen structural domain protein (FD), and the FD structural domain protein alone also has the same or a similar function as FGL1, wherein the amino acid sequence of Fibrinogen like protein 1 FGL1 is as shown in SEQ ID No. 1 in the sequence table, and the amino acid sequence of the FD structural domain protein alone is as shown at positions 48-290 in the sequence table in SEQ ID No. 1.

The amino acid sequence of Fibrinogen like protein 1 (FGL1) is shown below:

LEDCAQEQMRLRAQVRLLETRVKQQQVKIKQLLQENEVQFLDKGDENTVIDLGS
KRQYADCSEIFNDGYKLSGFYKIKPLQSPAEFSVYCDMSDGGGWTVIQRRSDGSENFNR
GWKDYENGFGNFVQKHGEYWLGNKNLHFLTTQEDYTLKIDLADFEKNSRYAQYKNFK
VGDEKNFYELNIGEYSGTAGDSLAGNFHPEVQWWASHQRMKFSTWDRDHDNYEGNC
AEEDQSGWWFNRCHSANLNGVYYSGPYTAKTDNGIVWYTWHGWWYSLKSVVMKIRP
NDFIPNVI

The amino acid sequence of the fibrinogen structural domain protein (FD) is shown below:

TVIDLGSKRQYADCSEIFNDGYKLSGFYKIKPLQSPAEFSVYCDMSDGGGWTVIQ
RRSDGSENFNRGWKDYENGFGNFVQKHGEYWLGNKNLHFLTTQEDYTLKIDLADFEK
NSRYAQYKNFKVGDEKNFYELNIGEYSGTAGDSLAGNFHPEVQWWASHQRMKFSTWD
RDHDNYEGNCAEEDQSGWWFNRCHSANLNGVYYSGPYTAKTDNGIVWYTWHGWWY
SLKSVVMKIRPNDFIPNVI

Specifically, in the Fibrinogen like protein 1 provided by the present invention, the amino acid sequence comprises tryptophan at position 204, tyrosine at position 222, cysteine at position 226, glutamic acid at position 229, aspartic acid at position 230, cysteine at position 239, histidine at position 240, aspartic acid at position 258, tyrosine at position 264, and tryptophan at position 269.

The amino acid sites listed in the above amino acid sequences are key amino acid sites that can bind to the substrate GPRP (Gly-Pro-Arg-Pro) peptide. The amino acid sequences can specifically and competitively bind to the substrate GPRP by hydrophobic bonding, hydrogen bonding, or salt bonding through these key amino acid sites, as shown in Figure 26, which competitively occupy the binding sites of the peptide that can bind to the fibrinopeptide A, thus achieving the effect of inhibiting thrombosis or of slowing down the progress of thrombosis by inhibiting the assembly of fibrin and inhibiting the activity of fibrin.

### Example 3:

The present invention also provides nucleic acid molecules encoding Fibrinogen like protein 1. The nucleic acid molecule is a gene; the gene is a DNA molecule described in any one of the following:
(b1) a DNA molecule is shown in SEQ ID No. 2 (i.e., SEQ ID No. 2) or positions 142-870 of SEQ ID No. 2 (i.e., SEQ ID No. 4);
(b2) A DNA molecule that hybridizes under stringent conditions to a DNA molecule defined by (b1) and encodes the protein;
(b3) A DNA molecule having more than 99%, more than 95%, more than 90%, more than 85% or more than 80% homology to a DNA sequence defined in (b1)-(b2) and encoding the protein.

The nucleic acid sequence of Fibrinogen like protein 1 (FGL1) is shown below:

ctcgaggactgtgcccaggagcagatgcggctcagagcccaggtgcgcctgcttgagacccgggtcaaacagcaacaggtc
aagatcaagcagcttttgcaggagaatgaagtccagttccttgataaaggagatgagaatactgtcattgatcttggaagcaagaggcagtat
gcagattgttcagagattttcaatgatgggtataagctcagtggattttacaaaatcaaacctctccagagcccagcagaattttctgtttattgtg
acatgtccgatggaggaggatggactgtaattcagagacgatctgatggcagtgaaaactttaacagaggatggaaagactatgaaaatgg
ctttggaaattttgtccaaaaacatggtgaatattggctgggcaataaaaatcttcacttcttgaccactcaagaagactacactttaaaaatcga
ccttgcagattttgaaaaaaatagccgttatgcacaatataagaatttcaaagttggagatgaaaagaatttctacgagttgaatattggggaat
attctggaacagctggagattcccttgcggggaattttcatcctgaggtgcagtggtgggctagtcaccaaagaatgaaattcagcacgtgg
gacagagatcatgacaactatgaagggaactgcgcagaagaagatcagtctggctggtggtttaacaggtgtcactctgcaaacctgaatg
gtgtatactacagcggcccctacacggctaaaacagacaatgggattgtctggtacacctggcatgggtggtggtattctctgaaatctgtgg
ttatgaaaattaggccaaatgattttattccaaatgtaatt

The nucleic acid sequence of the fibrinogen structural domain protein (FD) is shown below:

actgtcattgatcttggaagcaagaggcagtatgcagattgttcagagattttcaatgatgggtataagctcagtggattttacaaaa
tcaaacctctccagagcccagcagaattttctgtttattgtgacatgtccgatggaggaggatggactgtaattcagagacgatctgatggcag
tgaaaactttaacagaggatggaaagactatgaaaatggctttggaaattttgtccaaaaacatggtgaatattggctgggcaataaaaatctt
cacttcttgaccactcaagaagactacactttaaaaatcgaccttgcagattttgaaaaaaatagccgttatgcacaatataagaatttcaaagtt
ggagatgaaaagaatttctacgagttgaatattggggaatattctggaacagctggagattcccttgcggggaattttcatcctgaggtgcagt
ggtgggctagtcaccaaagaatgaaattcagcacgtgggacagagatcatgacaactatgaagggaactgcgcagaagaagatcagtctg
gctggtggtttaacaggtgtcactctgcaaacctgaatggtgtatactacagcggcccctacacggctaaaacagacaatgggattgtctggt
acacctggcatgggtggtggtattctctgaaatctgtggttatgaaaattaggccaaatgattttattccaaatgtaatt

The present invention also provides expression cassettes, recombinant vectors, recombinant bacteria or transgenic cell lines comprising the above nucleic acid molecules.

### Example 4:

The present invention provides an application of the above-described Fibrinogen like protein 1 or of the above-described nucleic acid molecules or the above-described recombinant vectors, recombinant bacteria, or transgenic cell line, the application being at least any one of the following:
(c1) preparation of products for inhibiting thrombogenesis;
(c2) Preparation of products for inhibiting the extent of thrombosis;
(c3) Preparation of products for inhibiting fibrin activity in plasma;
(c4) Preparation of products used to inhibit fibrinogen assembly in plasma;
(c5) Preparation of products used to enhance fibrinolytic capacity;
(c6) Preparation of products for prolonging activated partial thromboplastin time of plasma and/or prothrombin time and/or thrombin time.

The studies show that Fibrinogen like protein 1 (FGL1) or fibrinogen structural domain protein (FD) inhibits fibrin assembly during thrombosis, and can be used to prepare a drug for treating thrombosis-related diseases.

Wherein the drug for treating a thrombosis-related disease is a drug having Fibrinogen like protein 1 (FGL1) or fibrinogen structural domain protein (FD) as an active ingredient. The drug for the treatment of thrombosis-related disorders can be used for the treatment of thrombotic diseases caused by a variety of reasons, as well as for the prevention of thrombotic diseases, at the same time, and for the prevention and treatment of a variety of disorders associated with increased fibrin activity, decreased fibrinolytic capacity, and shortening of the plasma's activated partial thromboplastin time and/or plasminogen time and/or prothrombin time.

Fibrinogen like protein 1 (FGL1) comprises Fibrinogen structural domain proteins (FDs), and its mechanism of action is that it can inhibit fibrin assembly during thrombosis by competing for binding of the fibrin γ subunit to fibrinopeptide A.

Fibrinogen like protein 1 (FGL1) may also be mutated to increase its affinity for fibrinopeptide A, thereby improving the drug's efficacy.

The method of preparation of the protein is to clone the gene encoding the protein into a eukaryotic expression vector, culture eukaryotic cells to express the protein in serum-free medium, purify the protein by using a Ni-NTA affinity column, and then further purify the protein by gel filtration using a Superdex 200 10/300 GL chromatographic column, to obtain a highly purified protein.

The product is a pharmaceutical product, the pharmaceutical product comprises a vaccine product.

The type of drug product comprises a DNA drug, an mRNA drug, a protein drug or an adenovirus drug. The drug product for the prevention or treatment of thrombotic diseases, which includes but is not limited to the types as mentioned above of products, may be administered in various forms of carriers, such as plasmid encapsulation, microsphere encapsulation, exosome carrying, etc., and may also be administered in various forms of protein-carrying delivery modes, such as protein-compound conjugation delivery, protein-peptide conjugation delivery, protein-small-molecule-drug conjugation delivery, etc., which ultimately aim to ensure the biological activity of the Fibrinogen like protein 1 (FGL1) or the fibrinogen structural domain protein (FD) described in the present invention, and ingesting the active ingredient through different delivery methods to inhibit the assembly of fibrin.

The product is modified with a structure for crossing the blood-brain barrier.

The structure modified for crossing the blood-brain barrier is a brain-targeting ligand.

The brain-targeting ligand includes but is not limited to RVG peptide, stearic acid-transferrin, and carrier protein E.

Based on the above-described description, on the premise of ensuring the biological activity of fibrinogen like protein 1 (FGL1) or fibrinogen structural domain protein (FD), the pharmaceutical product for preventing or treating thrombotic disease, can also be combined with a variety of targeting ligands. A more prominent targeting ligand modification is to combine the protein or the structural domain protein with the brain-targeting ligand to facilitate its breakthrough of the blood-brain barrier (BBB), thereby realizing supply of drugs to the organism for treatment for brain thrombosis. However, the present invention is not limited to this, as it can likewise be combined with other structures or forms of targeting ligands, thereby realizing targeted therapy for different lesions.

The drug also comprises a therapeutic agent that can be used in combination.

The combinable therapeutic agents are urokinase, streptokinase, alteplase, reteplase or tissue fibrinogen activator, respectively.

Fibrinogen like protein 1 (FGL1) and its mutants can effectively synergize with existing antithrombotic drugs (urokinase and tissue fibrinogen activator, etc.) and reduce the dose of existing antithrombotic drugs and alleviate their toxic side effects.

At the same time, the present invention provides a biomaterial, the biomaterial being a nucleic acid molecule capable of expressing the above-described protein or an expression cassette, a recombinant vector, a recombinant bacterium, or a transgenic cell line containing the nucleic acid molecule.

### Example 5:

Fibrinogen like protein 1 (FGL1) and fibrinogen structural domain (FD) inhibit fibrin aggregation:

### A, Preparation of Fibrinogen like protein 1 (FGL1):

The gene encoding the Fibrinogen like protein 1 (FGL1) protein was cloned into a eukaryotic expression vector, and eukaryotic cells were cultured in serum-free medium to express the said protein, which was purified by using a Ni-NTA affinity column. Then the protein was further purified by gel filtration using a Superdex 200 10/300 GL chromatographic column, to obtain the high purity of purified protein, which was verified with SDS- PAGE.

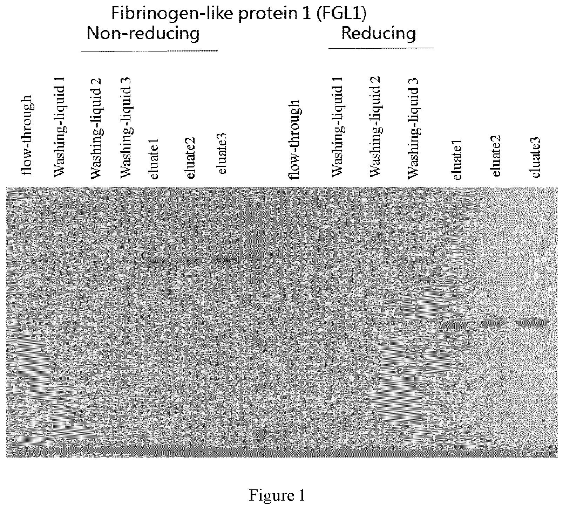

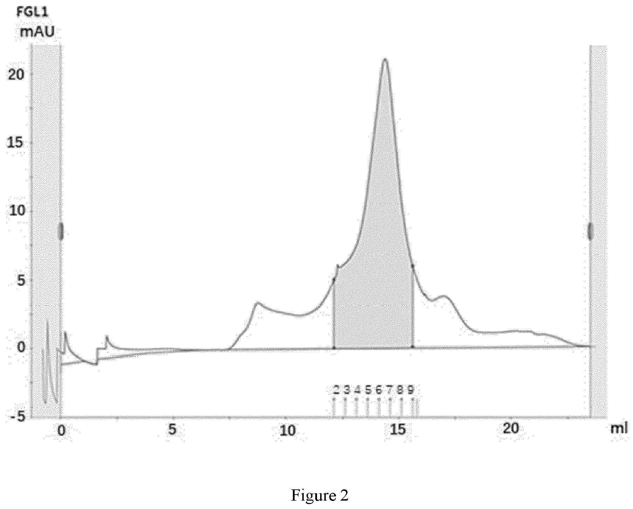

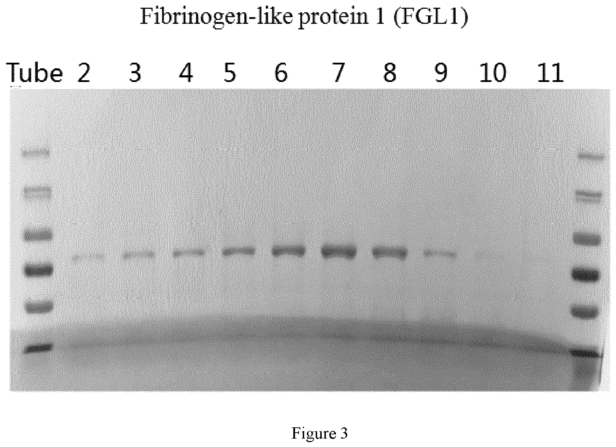

The SDS-PAGE photograph of Fibrinogen like protein 1 (FGL1) after purification by Ni-NTA affinity column is shown in Figure 1;
The elution plot of molecular sieve purification of the Fibrinogen like protein 1 (FGL1) is shown in Figure 2;
The SDS-PAGE photograph after molecular sieving of Fibrinogen like protein 1 (FGL1) is shown in Figure 3.

### b, Fibrinogen structural domain protein (FD) was obtained as follows:

The expression and purification of the fibrinogen structural domain protein (FD): the gene coding for Fibrinogen like protein 1 (FGL1) protein was used as a template, and the expression vector was obtained using PCR and homologous recombination. Eukaryotic cells were cultured in serum-free medium to express the proteins, which were purified using a Ni-NTA affinity column. Then the proteins were further purified by gel filtration using a Superdex 200 10/300 GL chromatographic column to obtain high-purity purified proteins, which was verified by SDS-PAGE.

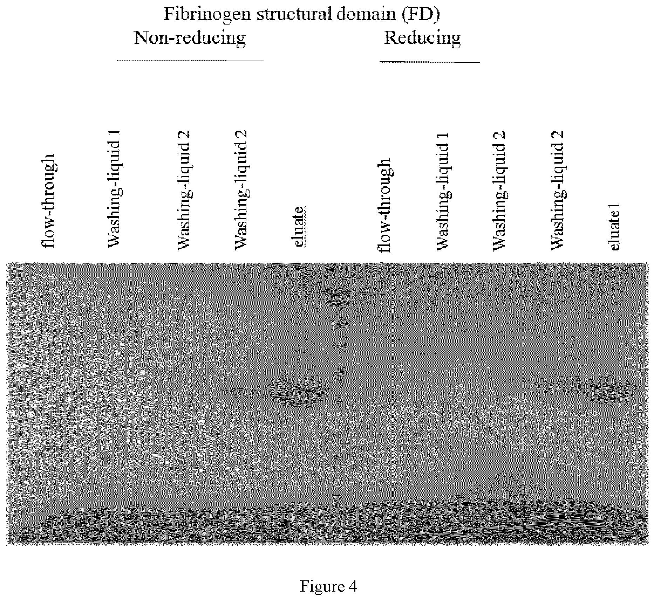

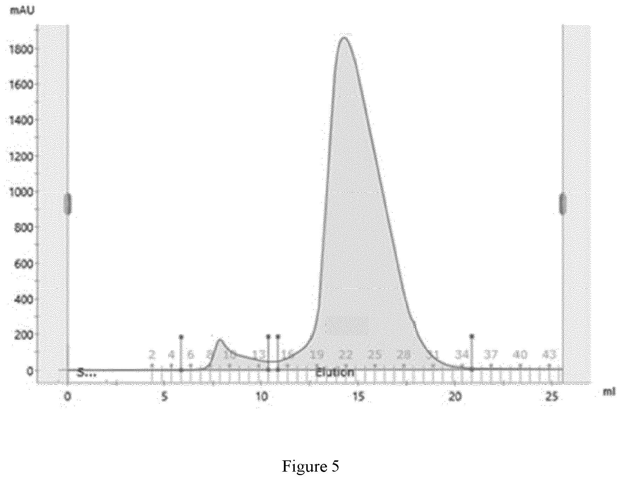

The SDS-PAGE photograph of the fibrinogen structural domain (FD) after purification by Ni-NTA affinity column is shown in Figure 4;
The elution plot of molecular sieve purification of the fibrinogen structural domain (FD) is shown in Figure 5;

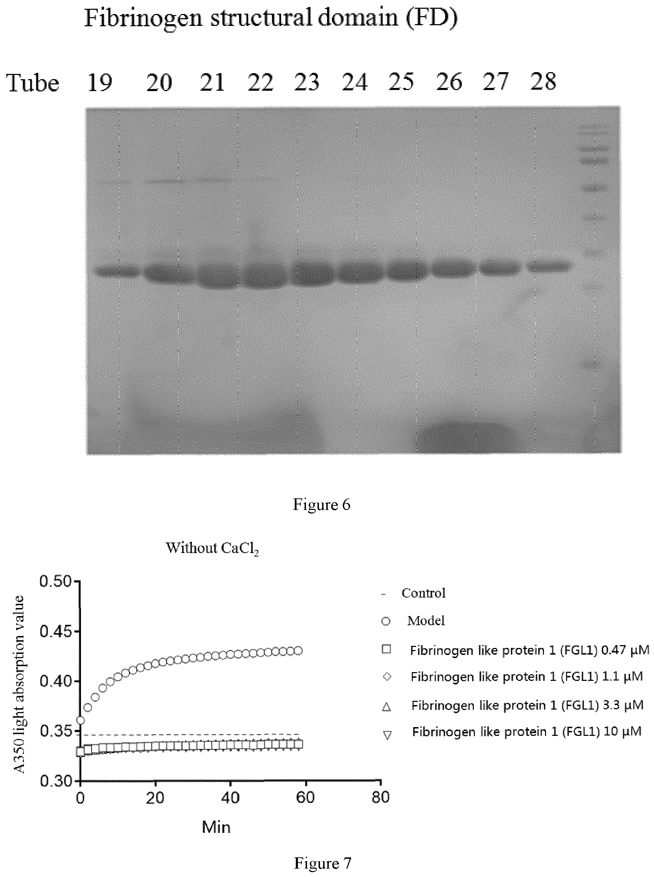

Figure 6 shows the SDS-PAGE photograph of the fibrinogen structural domain (FD) after molecular sieving.

### c, Fibrinogen like protein 1 (FGL1) inhibits fibrin aggregation:

Fibrinogen protein, also called coagulation factor I, is a hexameric complex composed of three subunits of α2β2γ2, an important thrombus component. Fibrin aggregation is mainly mediated by thrombin and coagulation factor 13. Coagulation factor 13 catalyzes the covalent linkage of amino acids in the C-terminal structural domain of the α or γ subunit. Thrombin cleaves the α and γ subunits of fibrin, exposing fibrinopeptide A (Knob A) and fibrinopeptide B (Knob B). The exposed fibrinopeptide A (Knob A) and fibrinopeptide B (Knob B) insert into the C-terminal structural domains of the γ and β subunits, respectively, of another fibrin molecule, thereby promoting fibrin aggregation. Although the tissue plasminogen activator tPA can directly target and degrade fibrin, the effect of some inhibitory factors makes it used with large dosages in clinical practice, causing side effects mainly including neurotoxicity and bleeding. Studies have shown that the loose structure of fibrin helps to enhance the degradation of tissue plasminogen activator tPA. Fibrinogen like protein 1 (FGL1) can inhibit fibrin aggregation by targeting fibrin.

### The experimental methods were as follows:

Multifunctional enzyme marker M200PRO detected the light scattering process, and the instrument excitation light was adjusted to 350 nm and emission light to 350 nm. 3.3 µM of Fibrinogen in the control group, 3.3 µM of Fibrinogen and 2.5 U/ml of thrombin in the model group and 3.3 µM of Fibrinogen and 2.5 U/ml thrombin and different dose of Fibrinogen related protein 1 (FGL1). All the above systems were in 20 mM Tris-HCl, pH 7.4, 150 mM NaCl ± 5 mM CaCl₂ solution in a 100 µl/well (96-well plate). The kinetic curve was measured by assaying every minute at 25°C.

Figure 7 shows the effect of fibrinogen-like protein 1 (FGL1) on fibrin aggregation in the absence of calcium chloride.

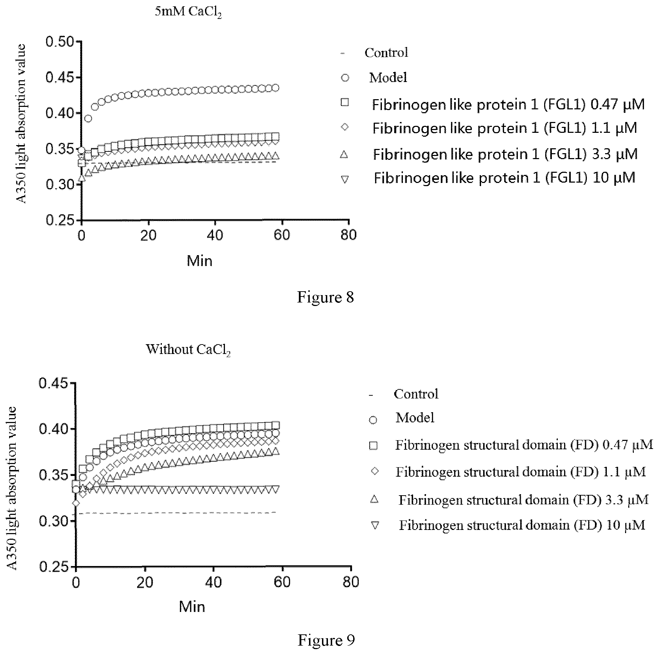

The effect of Fibrinogen like protein 1 (FGL1) on fibrin aggregation in the presence of calcium chloride is shown in Figure 8.

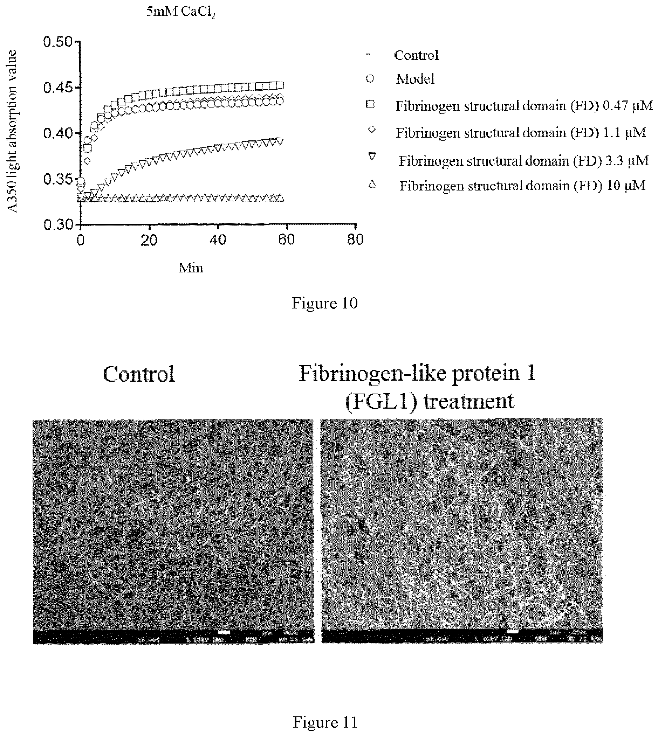

Figure 11 shows a comparative graph of the effect of fibrinogen like protein 1 (FGL1) on fibrin aggregation observed by scanning electron microscopy.

### d, Fibrinogen structural domain (FD) inhibits fibrin aggregation:

Similarly, fibrinogen structural domain (FD) can inhibit fibrin aggregation by targeting fibrin.

### The experimental methods were as follows:

Multifunctional enzyme marker M200PRO detected the light scattering process; the instrument excitation light was adjusted to 350 nm, and the emission light was adjusted to 350 nm. 3.3 µM of Fibrinogen in the control group, 3.3 µM of Fibrinogen and 2.5 U/ml of thrombin in the model group, and 3.3 µM of Fibrinogen and 2.5 U/ml thrombin and different dose of fibrinogen structural domain (FD). The above systems were in 20 mM Tris-HCl, pH 7.4, 150 mM NaCl ± 5 mM CaCl₂ solution. The kinetic curves were measured at 25°C by detecting the A350 absorption values every minute.

Figure 9 shows the effect of the fibrinogen structural domain (FD) on fibrin aggregation in the absence of calcium chloride.

The effect of fibrinogen structural domain (FD) on fibrin aggregation in the presence of calcium chloride is shown in Figure 10.

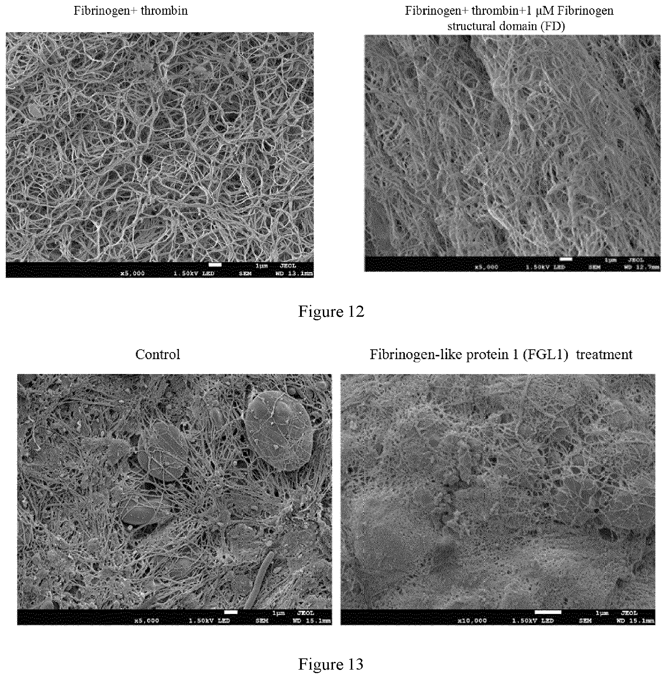

Figure 12 shows a comparative graph of the effect of fibrinogen structural domain (FD) on fibrin aggregation observed by scanning electron microscopy. The thrombus formed by fibrin aggregation becomes thinner and looser in structure under the treatment of fibrinogen structural domain (FD).

### Example 6:

### Protective effects of overexpression of Fibrinogen like protein 1 (FGL1) and fibrinogen structural domain (FD) plasmid injected in mice on thrombin-induced thrombosis model:

1. Protective effects of overexpression of Fibrinogen like protein 1 (FGL1) plasmid injected in mice on thrombin-induced thrombosis model:
   a. Grouping of mice:
      Female eight-week-old BALB/c mice (18-22 g) were randomly divided into 3 groups, i.e., normal control group, model group, and Fibrinogen like protein 1 (FGL1) plasmid injection group;
   **b.** Validation of Fibrinogen like protein 1 (FGL1) plasmid injection and overexpression system:
      Construct FGL1 expression plasmid with pcDNA3.1 vector, inject the plasmid into the tail vein of mice, 100 µg/2 ml PBS/per mouse, extract the plasma of the mice after 18 hours, and detect the overexpressed FGL1 content in the plasma by ELISA;
   c. Protective effects of Fibrinogen like protein 1 (FGL1) on thrombin-induced thrombosis model:

Thrombin 15 U/200 µl PBS/ per mouse was injected into the tail vein for modelling, and the number of mice in each group that died within 1 hour after injection was recorded to calculate the protection rate of Fibrinogen like protein 1 (FGL1) overexpression.

Figure 13 shows a comparative graph of the effect of fibrinogen like protein 1 (FGL1) on thrombus morphology in mice, as observed by scanning electron microscopy.

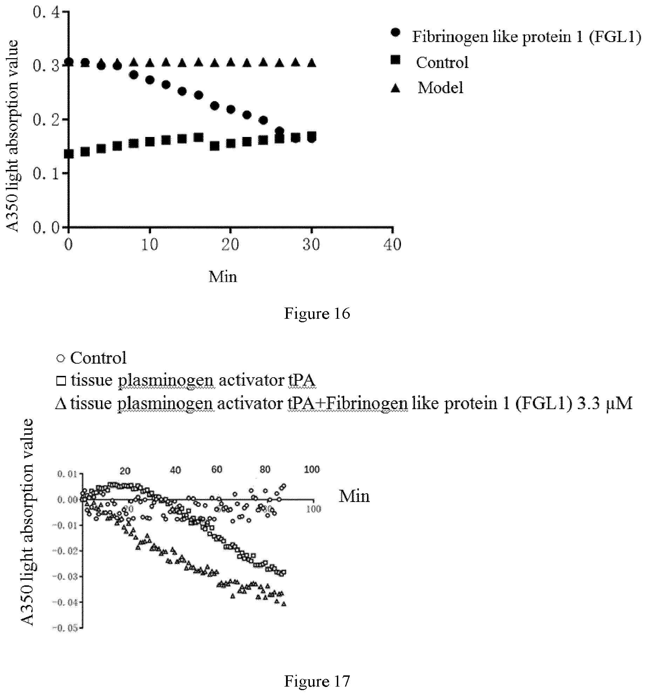

A control plot of the effect of action of Fibrinogen like protein 1 (FGL1) on the cleavage of aggregated fibrin is shown in Figure 16.

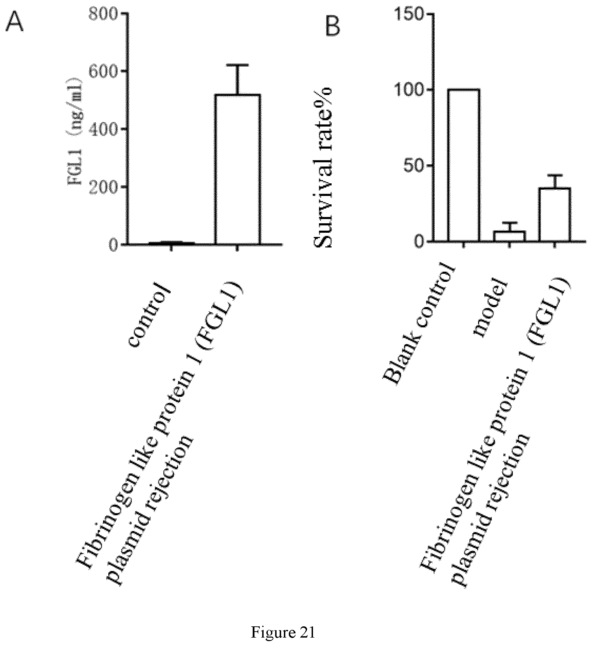

Statistical data of Fibrinogen like protein 1 (FGL1) plasmid injection in mice overexpressing improved thrombin-induced thrombus model are shown in Figure 21.

2, Protective effects of overexpression of fibrinogen structural domain (FD) plasmid injected in mice on thrombin-induced thrombosis model:
The specific operation methods were the same as those described previously for the protective effects of overexpression of FGL1.

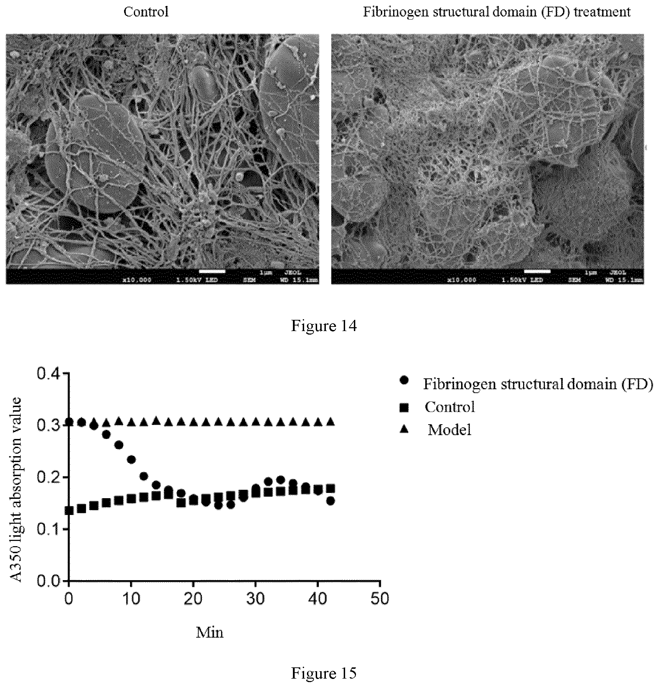

Figure 14 shows a scanning electron microscopy observation of the effect of fibrinogen structural domain (FD) on the morphology of mouse thrombus. Under the treatment of fibrinogen structural domain (FD), the thrombus becomes thinner and looser in structure.

A comparative graph of the effect of fibrinogen structural domain (FD) on the morphology of mouse thrombus observed by scanning electron microscopy, in which it can be seen that the thrombus becomes thinner and looser in structure under the treatment of fibrinogen structural domain (FD) is shown in Figure 14.

Figure 15 shows a control plot of the effect of the action of the fibrinogen structural domain (FD) on the cleavage of aggregated fibrin.

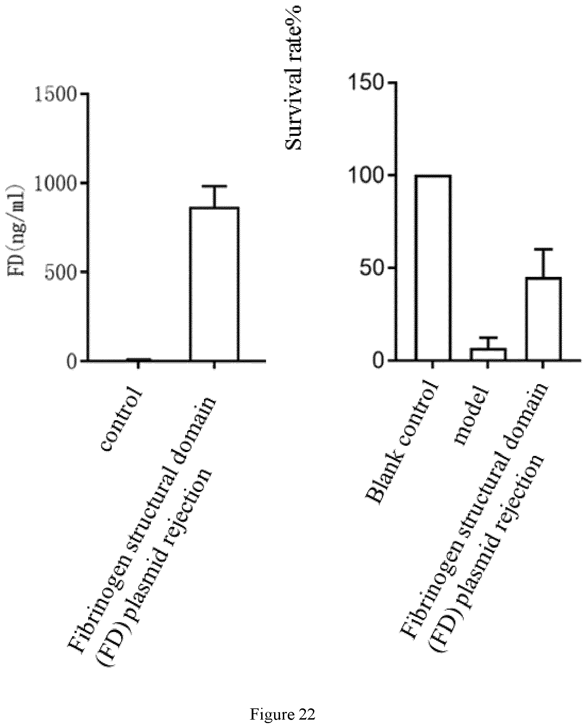

Figure 22 shows statistical graphs of data from the thrombin-induced thrombus model improved by overexpression of the fibrinogen structural domain (FD) plasmid injected into mice.

### Example 7:

### RVG peptide modification to promote entry of Fibrinogen like protein 1 (FGL1) into the mouse blood-brain barrier:

a. Test Material:
   RVG29- (YTWMPENPRPGTPCDIFTNSRGKRASNGGGGGGC) peptide was synthesized by Nanjing Genscript, a-maleimide-u-N-hydroxy-succinimide ester polyethylene glycol (MAL-PEG-NHS, molecular weight 2.1 kDa) was purchased from Creative PEGWorks, USA, and NHS-cy5.5 dye was purchased from Thermo Fisher;
b. Fibrinogen like protein 1 (FGL1) modified by MAL-PEG (MAL-PEG-FGL1) was prepared:
   MAL-PEG-NHS and Fibrinogen like protein 1 (FGL1) were reacted at a molar ratio of 1:2 in PBS (pH 8.0, 0.3 mL) for 3h at room temperature; 10 kDa concentration tubes were used to concentrate the product and to displace the unreacted MAL-PEG-NHS;
c. preparation of fibrinogen like protein 1 (FGL1) modified by RVG29-Cys-PEG (RVG29-Cys-PEG-FGL1):
   RVG29-Cys peptide and MAL-PEG-FGL1 were reacted at a molar ratio of 5:1 in PBS (pH 7.0, 0.3 mL) for 24 h at room temperature; 10 kDa concentration tubes were used to concentrate the product and to displace unreacted RVG29-Cys peptide;
d. preparation of NHS-cy5.5-labeled RVG29-Cys-PEG-FGL1 protein:
   10 µg of NHS-cy5.5 and 10 mg of RVG29-Cys-PEG-FGL1 were reacted in PBS (pH 8.0, 0.3 mL) for 1 h at room temperature, and 10 kDa concentration tubes were used to concentrate the product and to displace unreacted NHS-cy5.5;
e. Observe the fluorescence content of NHS-cy5.5-labeled RVG29-Cys-PEG-FGL1 protein in brain tissue:
   Female eight-week-old BALB/c mice (18-22 g)were randomly divided into 3 groups, i.e., normal control group, NHS-cy5.5-labeled FGL1 group, and NHS-cy5.5-labeled RVG29-Cys-PEG-FGL1 group; mice were injected with 10 mg/kg of proteins in the tail vein, and then after 1, 2, and 6 hours, the distribution of proteins in organs of the mice was observed using a small animal imager (IVIS Lumina XR) to observe.

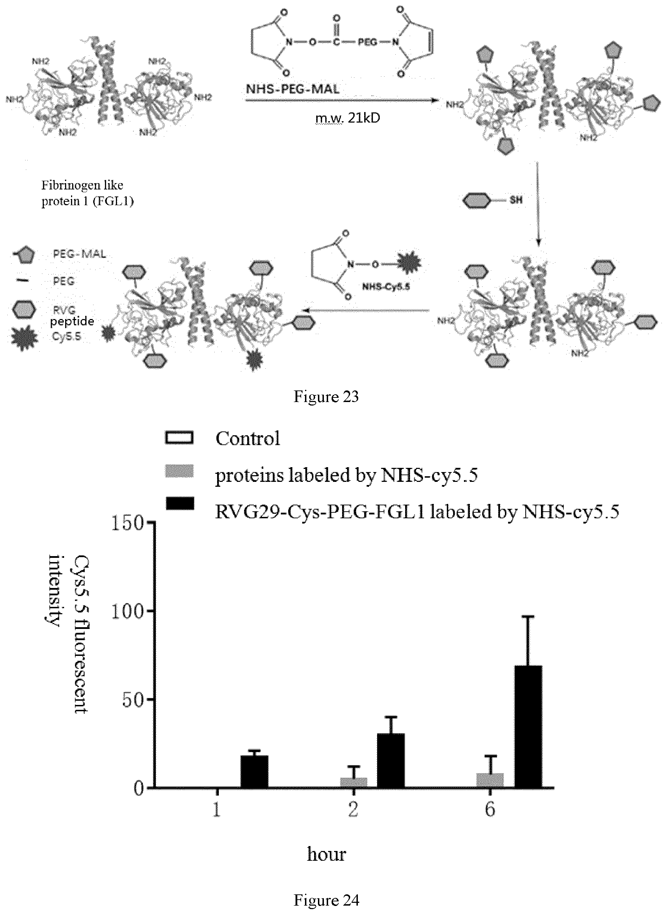

Figure 23 shows an operational procedure for Fibrinogen like protein 1 (FGL1) modified by RVG peptide and Cy5.5 fluorescence.

A graph of the results of the distribution assay of Fibrinogen like protein 1 (FGL1) in mouse brain tissue promoted by modification of RVG peptide is shown in Figure 24.

### Example 8:

### Synergistic use of Fibrinogen like protein 1 (FGL1) with tissue plasminogen activator tPA:

a. Synergistic use of Fibrinogen like protein 1 (FGL1) with tissue plasminogen activator tPA promoting fibrin aggregation:
   The experimental methods were as follows:
   The Multifunctional enzyme marker M200PRO detected the light scattering process, and the excitation light of the instrument was adjusted to 350 nm and the emission light to 350 nm. 3.3 µM of Fibrinogen in the control group, 3.3 µM of Fibrinogen and 2.5 U/ml of thrombin in the model group. The above systems were all set up in 20 mM Tris-HCl, pH 7.4, 150 mM NaCl ± 10 mM CaCl₂ solution in a 100 µl/well (96-well plate). After 1 h of gel-like fibrin formation, 100 µl, 2 nM tissue plasminogen activator tPA and different doses of Fibrinogen like protein 1 (FGL1) were added. Kinetic curves were measured at 25°C by detecting A350 absorption values every minute.
b. Synergistic use of Fibrinogen like protein 1 (FGL1) with the tissue plasminogen activator tPA promoting thrombolysis:
   Methods were as follows:
   1) Preparation of thrombus:
      Freshly prepared thrombus samples were obtained by first adding 5 µl of promoting-thrombotic drop (Innovin + CaCl₂) along the bottom of the well wall in a 96-well plate, followed by adding 25 µl of fresh mouse blood to the bottom of the well wall. The systems were incubated at 37°C for 30 minutes.
   2) Lysis of the thrombus:
      The control group was a reagent control, and the administered groups were 2 nM tissue plasminogen activator tPA, different doses of Fibrinogen like protein 1 (FGL1) alone groups, and groups in combination with other reagents. Kinetic curves were measured at 37°C using a multifunctional enzyme marker M200PRO to detect the absorption values of the 510 nm excitation light every minute.

Figure 17 shows a comparative plot of fibrinogen like protein 1 (FGL1) synergistically dissolving fibrin aggregates with tissue fibrinogen activator tPA.

### Example 9:

### Synergistic use of fibrinogen structural domain (FD) with tissue plasminogen activator tPA:

a. Synergistic use of fibrinogen structural domain (FD) with tissue plasminogen activator tPA promoting fibrin aggregation:
   The experimental methods were as follows:
   The Multifunctional enzyme marker M200PRO detected the light scattering process, and the excitation light of the instrument was adjusted to 350 nm and the emission light to 350 nm. 3.3 µM of Fibrinogen in the control group, 3.3 µM of Fibrinogen and 2.5 U/ml of thrombin in the model group. The above systems were all set up in 20 mM Tris-HCl, pH 7.4, 150 mM NaCl ± 10 mM CaCl₂ solution in a 100 µl/well (96-well plate). After 1 h of gel-like fibrin formation, 100 µl, 2 nM tissue plasminogen activator tPA and different fibrinogen structural domain (FD) doses were added. Kinetic curves were measured at 25°C by detecting A350 absorption values every minute.
b. Synergistic use of fibrinogen structural domain (FD) with the tissue plasminogen activator tPA promoting thrombolysis:
   Methods were as follows:
   1) Preparation of thrombus:
      Freshly prepared thrombus samples were obtained by first adding 5 µl of promoting-thrombotic drop (Innovin + CaCl₂) along the bottom of the well wall in a 96-well plate, followed by adding 25 µl of fresh mouse blood to the bottom of the well wall. The systems were incubated at 37°C for 30 minutes.
   2) Lysis of the thrombus:
      The control group was a reagent control, and the administered groups were 2 nM tissue plasminogen activator tPA, different doses of fibrinogen structural domain (FD) alone groups, and groups in combination with other reagents. Kinetic curves were measured at 37°C using a multifunctional enzyme marker M200PRO to detect the absorption values of the 510 nm excitation light every minute.

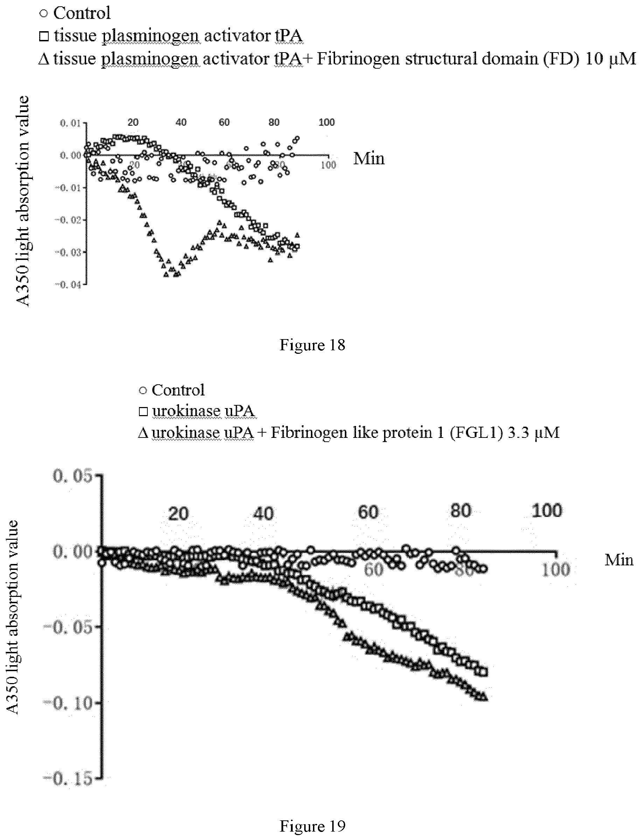

A comparative plot of fibrinogen structural domain (FD) synergistically dissolving fibrin aggregates with tissue fibrinogen activator tPA is shown in Figure 18.

### Example 10:

### Synergistic use of Fibrinogen like protein 1 (FGL1) with urokinase uPA:

a. Synergistic use of Fibrinogen like protein 1 (FGL1) with urokinase uPA promoting fibrin aggregation:
   The experimental methods were as follows:
   The Multifunctional enzyme marker M200PRO detected the light scattering process, and the excitation light of the instrument was adjusted to 350 nm and the emission light to 350 nm. 3.3 µM of Fibrinogen in the control group, 3.3 µM of Fibrinogen and 2.5 U/ml of thrombin in the model group. The above systems were all set up in 20 mM Tris-HCl, pH 7.4, 150 mM NaCl ± 10 mM CaCl₂ solution in a 100 µl/well (96-well plate). After 1 h of gel-like fibrin formation, 100 µl, 800 U/ml urokinase uPA and different doses of Fibrinogen like protein 1 (FGL1) were added. Kinetic curves were measured at 25°C by detecting A350 absorption values every minute.
b. Synergistic use of Fibrinogen like protein 1 (FGL1) with the urokinase uPA promoting thrombolysis:
   Methods were as follows:
   1) Preparation of thrombus:
      Freshly prepared thrombus samples were obtained by first adding 5 µl of promoting-thrombotic drop (Innovin + CaCl₂) along the bottom of the well wall in a 96-well plate, followed by adding 25 µl of fresh mouse blood to the bottom of the well wall. The systems were incubated at 37°C for 30 minutes.
   2) Lysis of the thrombus:
      The control group was a reagent control, and the administered groups were 800 U/ml urokinase uPA, different doses of Fibrinogen like protein 1 (FGL1) alone groups, and groups in combination with other reagents. Kinetic curves were measured at 37°C using a multifunctional enzyme marker M200PRO to detect the absorption values of the 510 nm excitation light every minute.

A comparative plot of Fibrinogen like protein 1 (FGL1) synergistically dissolving fibrin aggregates with urokinase uPA is shown in Figure 19.

### Example 11:

Synergistic use of fibrinogen structural domain (FD) with urokinase uPA:
a. Synergistic use of fibrinogen structural domain (FD) with urokinase uPA promoting fibrin aggregation:
   The experimental methods were as follows:
   The Multifunctional enzyme marker M200PRO detected the light scattering process, and the excitation light of the instrument was adjusted to 350 nm and the emission light to 350 nm. 3.3 mM of Fibrinogen in the control group, 3.3 mM of Fibrinogen and 2.5 U/ml of thrombin in the model group. The above systems were all set up in 20 mM Tris-HCl, pH 7.4, 150 mM NaCl ± 10 mM CaCl₂ solution in a 100 µl/well (96-well plate). After 1 h of gel-like fibrin formation, 100 µl, 2800 U/ml urokinase uPA and different doses of fibrinogen structural domain (FD) were added. Kinetic curves were measured at 25°C by detecting A350 absorption values every minute.
b. Synergistic use of fibrinogen structural domain (FD) with the urokinase uPA promoting thrombolysis:
   Methods were as follows:
   1) Preparation of thrombus:
      Freshly prepared thrombus samples were obtained by first adding 5 µl of promoting-thrombotic drop (Innovin + CaCl₂) along the bottom of the well wall in a 96-well plate, followed by adding 25 µl of fresh mouse blood to the bottom of the well wall. The systems were incubated at 37°C for 30 minutes.
   2) Lysis of the thrombus:
      The control group was a reagent control, and the administered groups were 800 U/ml urokinase uPA, different doses of fibrinogen structural domain (FD) alone groups, and groups in combination with other reagents. Kinetic curves were measured at 37°C using a multifunctional enzyme marker M200PRO to detect the absorption values of the 510 nm excitation light every minute.

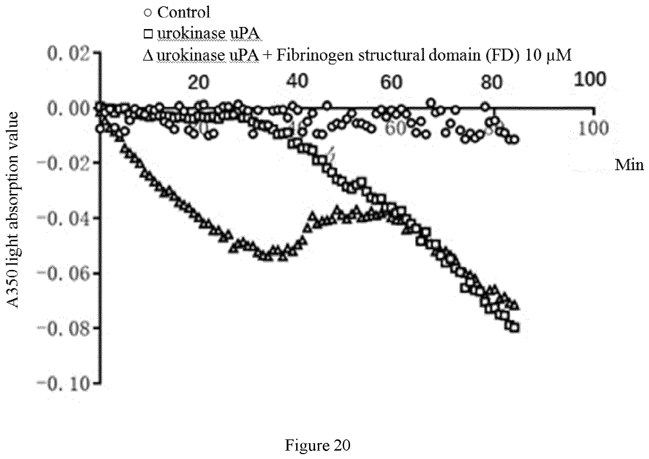

Figure 20 shows a comparative plot of fibrinogen structural domain (FD) synergistically dissolving fibrin aggregates with urokinase uPA.

### Example 12:

### Fibrinogen like protein 1 (FGL1) and fibrinogen structural domain (FD) have low toxic side effects:

Sixty female eight-week-old BALB/c mice (18-22 g) were randomly divided into five groups, i.e., normal control, Fibrinogen like protein 1 (FGL1) 100 mg/kg, Fibrinogen like protein 1 (FGL1) 30 mg/kg fibrinogen structural domain (FD) 100 mg/kg, fibrinogen structural domain (FD) 30 mg/kg and alteplase 10 mg/kg, tail vein administration was administered for 1 hr. Mice were anaesthetized with sodium pentobarbital, and bleeding time was measured. The tails were severed at 5 mm from the tip of the mouse's tail, while a stopwatch was used for timing. Every 10 s, a clean piece of filter paper was used to wipe the severed tail until no blood was stained on the filter paper, then the timing was stopped, and the time taken was recorded. The time was recorded as the bleeding time of the mouse tail. 2 hours later, plasma was extracted from the mice, and the plasma fibrinogen content was determined.

Fibrinogen like protein 1 (FGL1) and the structural domain of fibrinogen structural domain fibrinogen structural domain (FD) had no significant effect on the loss of Fibrinogen (A) and bleeding time (B) (no toxic side effects), and the results are shown in Figure 25.

### Example 13:

Mutation of certain key sites in this protein's amino acid sequence can also enhance its function of inhibiting thrombosis. In this embodiment, the tryptophan mutation at the key sites of position 204 and 269 was mutated to alanine, and then the method of Example 6 was applied to the mutated fibrinogen structural domain (FD), and comparison of the cleavage effect of the obtained fibrinogen structural domain (FD) mutant on aggregated fibrin is shown in Figure 27; as can be seen from the comparison results in Figure 27, the cleavage of aggregated fibrin by the mutant was significantly enhanced compared to that in the unmutated form.

Finally, it should be noted that the above embodiments are only used to illustrate the technical solution of the present invention and are not intended to be a limitation thereof. However, the present invention has been described in detail concerning the preceding examples, it should be understood by the person skilled in the art that: it is still possible to make modifications to the technical solution contained in the various preceding examples or to make equivalent substitutions for some or all of the technical features therein; and such modifications or substitutions that does not take the essence of the corresponding technical solutions away from the scope of the technical solutions of the various examples of the present invention.

## Claims

1. A protein, **characterized in** the said protein containing an amino acid sequence that can bind to a substrate GPRP peptide, wherein the structure of the amino acid sequence is: tryptophan-AAn1 -tyrosine-AAn2 -cysteine -AAn3-glutamic acid-aspartic acid-AAn4-cysteine-histidine-AAn5- Aspartic acid -AAn6-tyrosine -AAn7-tryptophan, wherein AA is an amino acid, n1-n7 are the number of amino acids, n1 = 16-18, n2=2-4, n3=1-3, n4=7-9, n5= 16-18, n6=4-6, n7=3-5; preferably, said number of amino acids n1= 17, n2= 3, n3= 2, n4= 8, n5= 17, n6= 5, n7= 4.

2. Fibrinogen like protein 1, **characterized in**, said Fibrinogen like protein 1 is any one of the following protein and comprising the amino acid sequence of claim 1:
(a1) A protein having the amino acid sequence as shown in SEQ ID No. 1 or positions 48-290 of SEQ ID No. 1;
(a2) A protein having the amino acid sequence shown in SEQ ID No. 1 or positions 48-290 of SEQ ID No. 1 in which one or several amino acid residues have been substituted and/or deleted and/or added and has the same functions;
(a3) A protein having more than 99%, more than 95%, more than 90%, more than 85% or more than 80% homology with the amino acid sequence defined in any one of (a1)-(a2) and having the same functions;
(a4) A fusion protein obtained by joining tags at the N-terminus and/or C-terminus of a protein defined in any one of (a1) - (a3).

3. Nucleic acid molecules encoding the said Fibrinogen like protein 1 of claim 2, **characterized in** said nucleic acid molecule is a gene; said gene is any one of the following DNA molecule:
(b1) a DNA molecule is shown in SEQ ID No. 2 or positions 142-870 of SEQ ID No. 2;
(b2) a DNA molecule that hybridizes under stringent conditions to a DNA molecule defined by (b1) and encodes said protein;
(b3) a DNA molecule having more than 99%, more than 95%, more than 90%, more than 85%, or more than 80% homology to a DNA sequence defined in (b1) - (b2) and encoding said protein.
(b4) A DNA molecule encoding said protein described in (a3).

4. Expression cassettes, recombinant vectors, recombinant bacteria or transgenic cell lines comprising said nucleic acid molecules of claim 3.

5. An application of the protein of claim 1, the Fibrinogen like protein 1 of claim 2, or the nucleic acid molecules of claim 3, or the recombinant vectors, recombinant bacteria or transgenic cell line of claim 4, **characterized in**, said application being at least any one of the following:
(c1) preparation of products for inhibiting thrombogenesis;
(c2) Preparation of products for inhibiting the extent of thrombosis;
(c3) Preparation of products for inhibiting fibrin activity in plasma;
(c4) Preparation of products used to inhibit fibrinogen assembly in plasma;
(c5) Preparation of products used to enhance fibrinolytic capacity;
(c6) Preparation of products for prolonging activated partial thromboplastin time of plasma and/or prothrombin time and/or thrombin time.

6. The application of claim 5, **characterized in**, the preparation method of the protein is: cloning the encoding gene of the protein into a eukaryotic expression vector, culturing eukaryotic cells to express the protein in serum-free medium, purifying the protein by using a Ni-NTA affinity column, and then further purifying by gel filtration of a Superdex 200 10/300 GL chromatographic column, obtaining a highly purified protein.

7. The application of claim 5 or 6, **characterized in**, said product is a pharmaceutical product, comprising a vaccine product; preferably, the type of the pharmaceutical product comprises an mRNA drug, a DNA drug, a protein drug, or an adenovirus drug.

8. The application of claim 5 or 6, **characterized in**, said product is modified with a structure for crossing the blood-brain barrier; preferably, said structure modified for crossing the blood-brain barrier is a brain-targeting ligand; preferably, said brain-targeting ligand, includes but is not limited to, RVG peptide, stearic acid-transferrin, and carrier protein E.

9. The application of claim 8, **characterized in**, the said pharmaceutical product also comprises a therapeutic agent capable of being used in combination; preferably, said therapeutic agent capable of being used in combination is urokinase, streptokinase, alteplase, reteplase or tissue fibrinogen activator, respectively.

10. A biomaterial, **characterized in that** the biomaterial is a nucleic acid molecule capable of expressing the protein of claim 1 or the nucleic acid molecule capable of expressing Fibrinogen like protein 1 of claim 2 or an expression cassette, a recombinant vector, a recombinant bacterium, or a transgenic cell line comprising the nucleic acid molecule.
